(19) Europäisches Patentamt
European Patent Office
Office européen des brevets

(11) EP 3 500 346 B1

(12) EUROPEAN PATENT SPECIFICATION

(45) Date of publication and mention of the grant of the patent:
**15.04.2020 Bulletin 2020/16**

(21) Application number: **17740742.6**

(22) Date of filing: **18.07.2017**

(51) Int Cl.:
***A61Q 17/00*** *(2006.01)* ***A61K 8/35*** *(2006.01)*
***A01N 35/04*** *(2006.01)* ***A01N 43/40*** *(2006.01)*
***A61Q 5/00*** *(2006.01)* ***A61Q 5/12*** *(2006.01)*
***A61Q 5/02*** *(2006.01)* ***A61Q 19/10*** *(2006.01)*

(86) International application number:
**PCT/EP2017/068105**

(87) International publication number:
**WO 2018/033328 (22.02.2018 Gazette 2018/08)**

(54) **AN ANTIMICROBIAL COMPOSITION**
ANTIMIKROBIELLE ZUSAMMENSETZUNG
COMPOSITION ANTIMICROBIENNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.08.2016 PCT/CN2016/000472**
**04.10.2016 EP 16192161**

(43) Date of publication of application:
**26.06.2019 Bulletin 2019/26**

(73) Proprietors:
- **Unilever N.V.**
  **3013 AL Rotterdam (NL)**
  Designated Contracting States:
  **AL AT BE BG CH CZ DE DK EE ES FI FR GR HR HU IS IT LI LT LU LV MC MK NL NO PL PT RO RS SE SI SK SM TR**
- **Unilever PLC**
  **London, Greater London EC4Y 0DY (GB)**
  Designated Contracting States:
  **CY GB IE MT**

(72) Inventors:
- **CAWLEY, Andrew Kenneth**
  **Bebington**
  **Wiral CH63 3JW (GB)**
- **HOPTROFF, Michael, John**
  **Bebington**
  **Wirral CH63 3JW (GB)**
- **PU, Mingming**
  **Shanghai 200335 (CN)**

(74) Representative: **Tansley, Sally Elizabeth**
**Unilever PLC**
**Unilever Patent Group**
**Colworth House**
**Sharnbrook**
**Bedford**
**Bedfordshire MK44 1LQ (GB)**

(56) References cited:
**WO-A1-01/39735 WO-A2-2013/010706**

- **HALL M J ET AL: "the fractional inhibitory concentration (FIC) index as a measure of synergy", JOURNAL OF ANTIMICROBIAL CHEMOTHERAPY, OXFORD UNIVERSITY PRESS, GB, vol. 11, no. 5, 1 May 1983 (1983-05-01), pages 427-433, XP008091249, ISSN: 0305-7453, DOI: 10.1093/JAC/11.5.427**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 500 346 B1

## Description

### Field of the invention

This invention relates to an antimicrobial composition. The invention more particularly relates to a personal care composition e.g those for hair, body and face wash which provides anti-microbial efficacy. It more particularly relates to a cleansing composition for hair and scalp comprising actives that interact to provide synergistic antimicrobial efficacy for anti-dandruff benefits.

### Background of the invention

The invention relates to an anti-microbial composition useful for cleaning of any body part but especially suitable for hair and scalp. Hair care compositions generally provide cleansing or conditioning benefits or a combination of the two. Such compositions typically comprise one or more cleansing surfactants which generally aid in cleaning the hair and/or the scalp free of undesirable soil, particles, and fatty matter.

Additionally, anti-dandruff benefit has been provided through hair care compositions. Dandruff is an issue that affects many people globally. The condition is manifested by the shedding of clumps of dead skin cells from the scalp. These are white in colour and provide an aesthetically displeasing appearance. A factor that contributes to dandruff are certain members of the Malassezia yeasts. To combat these, anti-dandruff products have been developed in the form of hair cleansing shampoos. An example of a known anti-dandruff shampoo comprises sodium lauryl ether sulfate (an ethoxylated anionic surfactant) in combination with an anti-dandruff agent. Typical anti-dandruff agents used in hair care are metal pyrithione e.g zinc pyrithione (ZPTO), octopirox (piroctone olamine), azole antimicrobials (e.g. climbazole), selenium sulfide and combinations thereof.

While the problem of dandruff is mitigated to a large extent through use of the above actives in such shampoos, there is a need for enhancing the efficacy of these actives. The present inventors have found through a combination of exploring very many possible mechanisms by which the efficacy of one of the above actives (zinc pyrithione) can be enhanced together with extensive experiments on possible enhancer candidates that a certain compounds of the benzophenone class (e.g. benzophenone -1 and benzophenone -2) display such enhancing properties. The synergistic antimicrobial combination of this invention can also be formulated into products for skin cleansing e.g. body and face care.

Benzophenones have been known to act as sunscreen agents and used extensively in skin care compositions.

The present inventors are not aware of any prior disclosed or used composition which has a combination of the actives of the present invention nor is there any indication that they can be combined to provide synergistic antimicrobial activity.

It is thus an object of the present invention to provide for a personal care composition that exhibits synergistic antimicrobial activity as compared to the individual components.

### Summary of the invention

According to the first aspect of the present invention there is a synergistic antimicrobial composition comprising

(i) A polyvalent metal salt of pyrithione; and
(ii) A benzophenone compound selected from benzophenone-1 or benzophenone-2.

The second aspect of the present invention relates to a method of inhibiting the growth of *Malassezia furfur* comprising the step of applying a composition as claimed in the first aspect, on the scalp of an individual.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are

described in the format "from x to y", it is understood that all ranges combining the different endpoints are also contemplated. In other words, in specifying any ranges of values, any particular upper value can be associated with any particular lower value.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

By 'an antimicrobial composition' as used herein, is meant to include a composition for topical application to skin, hair and/or scalp of mammals, especially humans. Such a composition is generally applied on to the desired topical surface of the body for a period of time from a few seconds to up to 24 hours. When the period of time of application is low say of the order of a few seconds to a few minutes after which the composition is rinsed off with water or wiped away, such a composition is known as a cleansing composition or a wash-off composition. When the composition is applied for longer period of time say from several minutes to up to 24 hours and washed off usually during the process of normal personal cleaning, such a composition is known as a leave-on composition. The composition as per the present invention may be of the wash-off or of the leave-on type. Of the two, it is preferably of the wash-off type. It includes any product applied to a human body for also improving appearance, cleansing, odor control or general aesthetics. The composition of the present invention can be in the form of a liquid, lotion, cream, foam, scrub, gel, or bar. Non-limiting examples of such compositions include wash-off shampoos, conditioners, shower gels, hand wash liquids or gels or bars. The composition of the present invention is most preferably a shampoo or a conditioner. It is most preferably used for preventing or alleviating the symptoms of dandruff on the scalp and/or hair.

The present invention more particularly relates to an antimicrobial cleansing composition. A preferred aspect is an anti-dandruff hair care composition. It comprises synergistic anti-fungal action of the polyvalent metal salt of pyrithione e.g. zinc pyrithione with the benzophenone compound claimed in the present invention.

The preferred polyvalent metal salt of pyrithione is zinc pyrithione (ZPTO) which is shorthand for zinc 1-hydroxy-2-pyridinethione.

The polyvalent metal salt of pyrithione is represented by the following general formula:

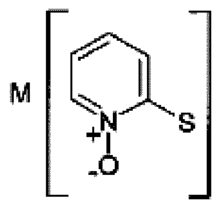

In the case of zinc pyrithione, M is the metal cation zinc.

Zinc pyrithione is preferably present in 0.1 to 5.0%, more preferably from 0.1 to 2.0% based on weight of the composition. ZPTO is a particulate material. While the particle size is not critical to achieve the benefits of the present invention, the particle size of ZPTO is preferably from 0.25 to 8 micrometer, more preferably from 0.5 to 8.0 micrometer, and further more preferably from 1.0 to 7.5 micrometer. ZPTO is commercially available from Kolon Life Science Inc., Sino Lion (USA) Ltd, Lonza, and other suppliers.

The benzophenone compound is preferably chosen from benzophenone -1 or benzophenone-2 which have the chemical structures given below.

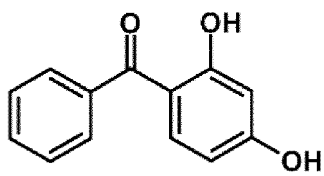

Benzophenone - 1

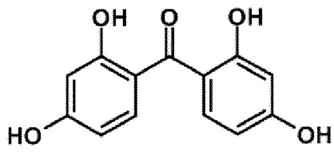

Benzophenone - 2

Benzophenone -1 and -2 are commercially available from Sigma-Aldrich Co. LLC., and other suppliers. Benzophenones absorb and dissipate UV radiation. In cosmetics and personal care products, they are used as sunscreens. In certain of those products such as lip balm and nail polish they are used to protect the products from UV light.

The benzophenone compound is preferably present in 0.1 to 10%, more preferably from 0.1 to 5%, further more preferably 0.2 to 3.0% based on the total weight of the composition. According to a preferred aspect of the present invention the benzophenone compound is benzophenone-1.

Without wishing to be bound by theory, the inventors believe that the anti-microbial efficacy of ZPTO is enhanced by the benzophenone compounds through perturbation of iron homeostasis.

The composition of the invention preferably comprises a cosmetically acceptable carrier. According to one aspect the cosmetically acceptable carrier comprises water. According to another preferred aspect, the carrier additionally comprises a surfactant.

According to a further preferred aspect of the present invention, the composition is either a shampoo or a hair conditioner.

Shampoo

As per an especially preferred aspect of the invention, the composition is a shampoo. The composition of the invention especially shampoos are formulated with an anionic surfactant e.g. an alkyl sulphate and/or ethoxylated alkyl sulfate surfactant. These anionic surfactants are preferably present at a level of from 1 to 20%, preferably 2 to 16%, further more preferably from 3 to 16% by weight of the composition. Preferred alkyl sulfates are C8-18 alky sulfates, more preferably C12-18 alkyl sulfates, preferably in the form of a salt with a solubilising cation such as sodium, potassium, ammonium or substituted ammonium.

Preferred alkyl ether sulfates are those having the formula: $RO(CH_2CH_2O)_nSO_3M$; wherein R is an alkyl or alkenyl having from 8 to 18 (preferably 12 to 18) carbon atoms; n is a number having an average value of greater than at least 0.5, preferably between 1 and 3, more preferably between 2 and 3; and M is a solubilising cation such as sodium, potassium, ammonium or substituted ammonium. An example is sodium lauryl ether sulfate (SLES). SLES having an average degree of ethoxylation of from 0.5 to 3, preferably 1 to 3 is especially preferred.

Shampoo compositions according to the invention may comprise one or more further anionic cleansing surfactants which are cosmetically acceptable and suitable for topical application to the hair.

Examples of further suitable anionic cleansing surfactants are the alkaryl sulphonates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in compositions of the invention include sodium oleyl succinate, ammonium lauryl sulphosuccinate, sodium lauryl ether sulphosuccinate, sodium dodecylbenzene sulphonate, triethanolamine dodecylbenzene sulphonate, lauryl ether carboxylic acid and sodium N-lauryl sarcosinate.

Suitable preferred additional anionic cleansing surfactants are sodium lauryl ether sulphosuccinate(n)EO, (where n is from 1 to 3), lauryl ether carboxylic acid (n) EO (where n is from 10 to 20).

Mixtures of any of the foregoing anionic cleansing surfactants may also be suitable.

A composition of the invention preferably additionally comprises an amphoteric surfactant preferably a betaine surfactant preferably an alkyl amidopropyl betaine surfactant for example cocamidopropyl betaine. In a preferred embodiment, the composition comprises from 0.1 to 10 wt.%, preferably from 0.5 to 8 wt.%, more preferably from 1 to 5 wt.% of a betaine surfactant

To enhance deposition of actives from compositions of the invention especially shampoos, cationic polymers are generally included therein. In the present invention too, it is preferred that the composition additionally includes 0.01 to 2.0% of a cationic polymer. The cationic polymer is preferably guar hydroxypropyl trimonium chloride. Guar polymer predominantly contains galactomannan polymer chains. This polymer is available at various molecular weights and degree of cationic substitutions depending on how much the guar has been hydrolysed and cationised. The cationic polymer is preferably present in 0.04 to 0.5%, more preferably 0.08 to 0.25% by weight of the composition.

The pH of the composition is preferably equal to or higher than 4.0, more preferably in the range of 5.0 to 7.0.

The composition as per the invention especially for anti-dandruff shampoos preferably additionally comprises a zinc compound. The presence of additional zinc compound in the composition is believed to improve the antidandruff efficacy of the metal salt of pyrithione. Suitable zinc compounds are zinc oxide, zinc citrate, zinc malonate, zinc carbonate or combinations thereof. The zinc compound is preferably present in 0.1 to 3%, more preferably 0.1 to 1.5% by weight of the composition.

Shampoo composition as per the invention preferably additionally comprises a conazole fungicide. Preferably the conazole fungicide is selected form ketoconazole, climbazole or mixtures thereof. The azole fungicide is preferably included in 0.01 to 2%, more preferably 0.025 to 0.75% by weight of the composition. The presence of a conazole fungicide is believed to improve the deposition of zinc pyrithione.

The composition preferably additionally comprises a vitamin B3 compound. The preferred vitamin B3 compound is niacinamide.

Niacinamide has the structure as given below

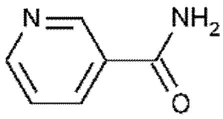

Niacinamide is known for secretion of AMPs from keratinocytes. The AMPs thus secreted provides for improving the immunity of the external surface of the body e.g. on the scalp. Thus with the use of niacinamide in the composition of the invention the anti-dandruff efficacy is expected to be enhanced not just through anti-fungal activity of the composition of the invention but by providing a boost to the scalp's own protection shield against germs, through use of niacinamide. It is expected that this combination could provide further long-lasting protection e.g. up to 24 hours of protection against germs.

Niacinamide is preferably present in 0.1 to 5%, more preferably 0.5 to 5%, further more preferably 0.5 to 3%, and optimally 1.0 to 3.0% by weight of the composition.

Suspending Agent

Preferably the composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent, if included, will generally be present in a shampoo composition of the invention at levels of from 0.1 to 10%, preferably from 0.5 to 6%, more preferably from 0.5 to 4% by total weight of suspending agent based on the total weight of the composition.

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

The composition of the invention is preferably aqueous based. It preferably comprises high amounts of water preferably from 70 to 95% by weight of the composition.

Hair Conditioner

When conditioning benefits are to be delivered through the composition of the invention the composition is

called a hair conditioner. Typically, the most popular conditioning agents used in hair care compositions are water-insoluble oily materials such as mineral oils, naturally occurring oils such as triglycerides and silicone polymers. Conditioning benefit is achieved by the oily material being deposited onto the hair resulting in the formation of a film, which makes the hair easier to comb when wet and more manageable when dry. An especially useful conditioning agent is a silicone compound, preferably a non-volatile silicone compound. Advantageously compositions herein may include one or more silicones. The silicones are conditioning agents found in dispersed or suspended particulate form. They are intended to deposit onto hair remaining behind after rinsing of the hair with water. Suitable silicone oils may include polyalkyl siloxanes, polyaryl siloxanes, polyalkylaryl siloxanes, polyether siloxane copolymers and mixtures thereof. Amino silicones are often formulated with shampoo compositions. Amino silicones are silicones containing at least one primary amine, secondary amine, tertiary amine or a quaternary ammonium group. High molecular weight silicone gums can also be utilized. Another useful type are the crosslinked silicone elastomers such as Dimethicone/Vinyl/Dimethicone Crosspolymers (e.g. Dow Corning 9040 and 9041).

Amounts of the silicone in compositions where present may range from about 0.1 to about 10 wt.%, preferably from about 0.1 to about 8wt.%, more preferably from about 0.3 to about 5wt.% by weight of the hair care compositions.

The pH of the composition is preferably equal to or higher than 4.0, more preferably in the range of 5.0 to 7.0.

The hair conditioning composition usually comprises conditioning surfactants selected from cationic surfactants, used singly or in admixture. Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant. Yet another preferred cationic surfactant is stearamidopropyl dimethylamine.

The most preferred cationic surfactants for use in the composition are stearamidopropyl dimethylamine, behentrimonium chloride, or stearyl trimethyl ammonium chloride. In conditioners of the invention, the level of cationic surfactant will generally range from 0.1 % to 5%, preferably 0.5 to 2.5% by weight of the composition.

Hair conditioning compositions of the invention preferably may also additionally comprise a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention will generally range from 0.5 to 10%, preferably from 0.1 % to 8%, more preferably from 0.2 % to 7 %, most preferably from 0.3 % to 6 % by weight of the composition. The weight ratio of cationic surfactant to fatty alcohol is suitably from 1:1 to 1:10, more preferably from 1:1.5 to 1:8, optimally from 1:2 to 1:5.

Skin cleansing

The composition of the invention may be used for skin care e.g. body or face wash. The antimicrobial composition may further comprise a surfactant. The preferred surfactants are nonionic surfactants, such as $C_8$-$C_{22}$, preferably $C_8$-$C_{16}$ fatty alcohol ethoxylates, comprising between 1 and 8 ethylene oxide groups when the product is in the liquid form. The surfactants are preferably selected from primary alkyl sulphate, secondary alkyl sulphonates, alkyl benzene sulphonates, or ethoxylated alkyl sulphates. The composition may further comprise an anionic surfactant, such as alkyl ether sulphate preferably those having between 1 and 3 ethylene oxide groups, either from natural or synthetic source and/or sulphonic acid. Especially preferred are sodium lauryl ether sulphates. Alkyl polyglucoside may also be present in the composition, preferably those having a carbon chain length between C6 and C16.

Thus, in a highly preferred aspect, the antimicrobial compositions include the surfactant selected from the group of anionic surfactant, fatty acid amide, alkyl sulphate, linear alkyl benzene sulphonate, and combinations thereof.

When the surfactants are present, the antimicrobial composition preferably comprises 1 to 90% surfactant by weight of the composition

When surfactant is used, a particularly preferred surfactant is soap. Soap is a suitable surfactant for personal washing applications of the antimicrobial composition of the invention. The soap is preferably $C_8$-$C_{24}$ soap, more preferably $C_{10}$-$C_{20}$ soap and most preferably $C_{12}$-$C_{18}$ soap. The cation of the soap can be alkali metal, alkaline earth metal or ammonium. Preferably, the cation of the soap is selected from sodium, potassium or ammonium. More preferably the cation of the soap is sodium or potassium.

A typical fatty acid blend consisted of 5 to 30% coconut fatty acids and 70 to 95% fatty acids by weight of soap. Fatty acids derived from other suitable oils/fats such as groundnut, soybean, tallow, palm, palm kernel, etc. may also be used in other desired proportions.

When present, the soap, of the present is preferably present in an amount of 1 to 90%, preferably from 10 to 85%, more preferably 25 to 75% by weight of the composition.

Preferred compositions may include other known ingredients such as perfumes, pigments, preservatives, emollients, sunscreens, emulsifiers, gelling agents and thickening agents. Choice of these ingredients will largely depend on the format of the composition.

Water is a preferred carrier. When water is present, it is preferably present in at least 1%, more preferably at least 2%, further more preferably at least 5% by weight of the composition. When water is the carrier, a preferred liquid composition comprises 10 to 99.8% by weight water. The liquid antimicrobial composition is useful as a skin antiseptic liquid, for skin cleansing, in particular for hand wash or a face wash. When water is the carrier, a preferred solid composition comprises 5 to 30% by weight water.

The solid antimicrobial composition is preferably in form of a shaped solid, more preferably a bar. The solid antimicrobial composition is particularly useful for skin cleansing in particular for hand wash or a face wash.

According to another aspect, inorganic particulate material is also a suitable carrier. When inorganic particulate material is the carrier, the antimicrobial composition is in a solid form. Preferably the inorganic particulate material is talc. When the inorganic particulate material is talc, the solid antimicrobial composition is particularly useful as a talcum powder for application on face or body.

According to a further aspect, a solvent is a preferred carrier. Although any solvent can be used, alcohol is a preferred solvent. Short chain alcohols, in particular ethanol and propanol, are particularly preferred as carrier for an antimicrobial wipe or an antimicrobial hand sanitizer composition.

In another aspect of the present invention, the composition of the present invention is suitable for use in wipes for personal hygiene.

The present invention also relates to a method of inhibiting the growth of *Malassezia furfur* comprising the step of applying a composition of the first aspect on to the scalp of an individual. Alternately and preferably the above step is followed by rinsing the surface with water. The step of rinsing is generally carried out within 1 to 5 minutes after application of the composition on the skin or hair/ scalp. According to one aspect, the invention provides for non-therapeutic benefits.

The method is more effective when the mole ratio of the polyvalent metal salt of pyrithione to the benzophenone compound on the *Malassezia furfur* cell is in the range of 1:20 to 1:2000.

The invention will now be illustrated with reference to the following non-limiting Examples.

**Examples**

The synergistic interaction between the ZPTO and the benzophenone compounds was determined using the DFIC assay. A brief description of the method is given below.

Methods: ΣFIC assay against *Malassezia furfur*

Microorganism preparation:

*M. furfur* (CBS 1878) was maintained on MD agar plate (Solution A) was cultured into 20ml of growth medium Pityrosporum Broth (PB, Solution B).

<u>Solution A preparation</u>

Modified Dixon Agar (MD)

36g Malt Extract (Oxoid)

(continued)

| | |
|---|---|
| 6g | Mycological Peptone (Oxoid) |
| 10 | Purified Agar (Oxoid) |
| 20g | Ox-bile (Oxoid) |
| 2ml | Oleic acid (Sigma) |
| 2ml | Glycerol (Sigma) |
| 10ml | Tween 40 (Sigma) |

Deionized Water to 1000ml
50mg (1 vial) dissolved in 2ml 95% ethanol Chloramphenicol (Oxoid SR078E)
(Ensure stirring thoroughly including after autoclaving)

Solution B preparation

Pityrosporum Broth (PB)

| | |
|---|---|
| 10g | Bacteriological Peptone |
| 0.1g | Yeast extract |
| 10g | Ox-bile |
| 2.5g | Taurocholic acid |
| 10g | Glucose |
| 1L | Deionised water |
| 0.5ml | Tween60 |
| 1ml | Glycerol |
| Adjust | pH to 6.2 |

After sterilization
0.5ml UHT milk

They are then incubated at 32 °C for 48 hrs with shaking. Then, 1ml of the first broth culture is transferred into 9ml of fresh PB and incubated at 32 °C for 48 hrs with shaking. The final culture should contain 2 to $6\times10^6$ cells/ml. This is achieved by diluting using PB to $5*10^5$ cell/ml.

In-vitro susceptibility testing

Zinc pyrithione was serially diluted (2-fold) to prepare in a range of 2-125 ppm in the growth medium. The benzophenone compound was serially diluted (2-fold) to prepare in a range of 10-10,000 ppm in growth medium.

Binary combinations of ZPTO and the testing compound were prepared in 96-well plate by mixing 20 $\mu$l of zinc pyrithione solution with 20 $\mu$l of testing compound. The solution in each well were further mixed with 160 $\mu$l of PB and 20 $\mu$l of M. *furfur.* The final cell density in the testing plate is around $5*10^4$ cell/ml, and the final concentration of actives in each well of the 96-wel plate were shown below.
ZPTO concentrations (in ppm): 12.5, 6.3, 3.1, 1.6, 0.8, 0.4, 0.2 and 0

Benzophenone compound concentrations (in ppm): 1000, 500, 250, 125, 62.5, 31.3, 15.6, 7.8, 3.9, 2.0, 1.0 and 0.

The plate was incubated at 32°C for one day. Then 20 $\mu$l of alamar blue (0.01%) was added into each well with a further incubation for one day to observe the color change. Red color indicates microbial growth and the non-changing blue color indicates no growth. In addition, it was also confirmed that the testing compound alone will not cause color change of alamar blue with a control mixture without adding microbes.

Calculation:

(1) Minimum Inhibition Concentration (MIC):

The MIC is defined as the absolute lowest concentration of active that provides complete microbial growth inhibition as indicated by the blue color of alamar blue under the tested condition. For example, for zinc pyrithione, the

MIC was determined to be 1.6-3.2 ppm.

The MIC of benzophenone - 1 and benzophenone - 2 was determined to be 500 ppm, and 1000 ppm respectively.

(2) Fractional Inhibition Concentration (FIC):

The differing behaviours of inhibitory antimicrobials in isolation and mixtures have been widely explored using the concept of the Fractional Concentration (FC) and Fractional Inhibitory Concentration (FIC). The parameter can be defined as follows:

$$\text{FIC (component a)} = \frac{\text{MIC (component a tested in the mixture)}}{\text{MIC (component a tested as a single active)}}$$

(3) Synergy and Additivity

The interactions between antimicrobials can be additive, synergistic or antagonistic depending on whether the efficacy of the combination is equivalent to, greater than or less than that obtained for the same total concentration of the individual components when tested alone.

These relationships can be expressed mathematically by summing the fractional MIC values for all the components present in the mixture to give the "fractional inhibition index". There is no consistent approach in the academic or patent literature in defining precise limiting ☐FIC values that differentiate synergy from additivity or antagonism. In this study, we have adopted a liberal approach defining any binary mixture with ☐FIC < 0.9 as showing evidence of synergistic behavior.

☐FIC = FIC (component 1) + FIC (component 2)
☐FIC = 1 corresponds to additive bactericidal activity
☐FIC > 1 corresponds to antagonistic bactericidal activity
☐FIC < 0.9 corresponds to synergistic bactericidal activity

4. Results:

Based on the concentration range of ZPTO (0 to 12.5 ppm) and the benzophenone compound (0 to 1000 ppm) at which the experiments were carried out, the ☐FIC of the combinations is given below.

| Example | Combination | ☐FIC |
|---|---|---|
| 1 | ZPTO + Benzophenone-1 | 0.32 |
| 2 | ZPTO + Benzophenone - 2 | 0.63 |

The data in Table above indicates that both the benzophenone compounds in combination with ZPTO exhibit synergistic antimicrobial activity.

## Claims

**1.** A synergistic antimicrobial composition comprising

(i) A polyvalent metal salt of pyrithione; and
(ii) A benzophenone compound selected from benzophenone-1 or benzophenone-2.

**2.** A composition as claimed in claim 1 wherein the polyvalent metal salt of pyrithione is zinc pyrithione.

**3.** A composition as claimed in claim 1 or 2 comprising 0.1 to 5.0% polyvalent metal salt of pyrithione.

**4.** A composition as claimed in any one of the preceding claims comprising 0.1 to 10% benzophenone compound by weight of the composition.

**5.** A composition as claimed in any one of the preceding claims wherein the benzophenone compound is benzophenone-1.

**6.** A composition as claimed in any one of the preceding claims wherein the composition comprises a cosmetically acceptable carrier comprising water.

**7.** A composition as claimed in claim 6 wherein the carrier additionally comprises a surfactant.

**8.** A composition as claimed in any one of the preceding claims wherein the composition is a wash-off or leave-on hair care composition.

**9.** A composition as claimed in claim 8 which is a shampoo or a hair conditioning composition.

**10.** A method of inhibiting the growth of Malassezia furfur comprising the step of applying a composition as claimed in any one of the preceding claims on the scalp of an individual.

**Patentansprüche**

**1.** Synergistische antimikrobielle Zusammensetzung, umfassend

(i) ein mehrwertiges Metallsalz von Pyrithion; und
(ii) eine unter Benzophenon-1 oder Benzophenon-2 ausgewählte Benzophenon-Verbindung.

**2.** Zusammensetzung, wie im Anspruch 1 beansprucht, wobei das mehrwertige Metallsalz von Pyrithion Zinkpyrithion ist.

**3.** Zusammensetzung, wie im Anspruch 1 oder 2 beansprucht, umfassend 0,1 bis 5,0% mehrwertiges Metallsalz von Pyrithion.

**4.** Zusammensetzung, wie in irgendeinem der vorhergehenden Ansprüche beansprucht, umfassend 0,1 bis 10% Benzophenon-Verbindung, nach Gewicht der Zusammensetzung.

**5.** Zusammensetzung, wie in irgendeinem der vorhergehenden Ansprüche beansprucht, wobei die Benzophenon-Verbindung Benzophenon-1 ist.

**6.** Zusammensetzung, wie in irgendeinem der vorhergehenden Ansprüche beansprucht, die einen Wasser enthaltenden kosmetisch verträglichen Träger umfasst.

**7.** Zusammensetzung, wie im Anspruch 6 beansprucht, wobei der Träger zusätzlich ein Tensid umfasst.

**8.** Zusammensetzung, wie in irgendeinem der vorhergehenden Ansprüche beansprucht, wobei die Zusammensetzung eine Abwasch- oder Leave-on-Haarpflegezusammensetzung ist.

**9.** Zusammensetzung, wie im Anspruch 8 beansprucht, welche ein Shampoo oder eine Haarkonditionierungszusammensetzung ist.

**10.** Verfahren zum Inhibieren des Wachstums von *Malassezia furfur*, umfassend den Schritt des Auftragens einer Zusammensetzung, wie in irgendeinem der vorhergehenden Ansprüche beansprucht, auf die Kopfhaut eines Individuums.

**Revendications**

**1.** Composition antimicrobienne synergique comprenant

(i) un sel de métal polyvalent de pyrithione ; et
(ii) un composé de benzophénone choisi parmi la benzophénone-1 ou benzophénone-2.

**2.** Composition selon la revendication 1, dans laquelle le sel de métal polyvalent de pyrithione est la pyrithione de zinc.

**3.** Composition selon la revendication 1 ou 2 comprenant de 0,1 à 5,0 % de sel de métal polyvalent de pyrithione.

**4.** Composition selon l'une quelconque des revendications précédentes comprenant de 0,1 à 10 % de composé de benzophénone en masse de la composition.

**5.** Composition selon l'une quelconque des revendications précédentes, dans laquelle le composé de benzophénone est la benzophénone-1.

**6.** Composition selon l'une quelconque des revendications précédentes, dans laquelle la composition comprend un support cosmétiquement acceptable comprenant de l'eau.

**7.** Composition selon la revendication 6, dans laquelle le support comprend de plus un tensioactif.

**8.** Composition selon l'une quelconque des revendications précédentes, dans laquelle la composition est une composition de soin des cheveux avec rinçage ou sans rinçage.

**9.** Composition selon la revendication 8 qui est un shampoing ou une composition d'après-shampoing.

**10.** Procédé d'inhibition de la croissance de *Malassezia furfur* comprenant l'étape d'application d'une composition selon l'une quelconque des revendications précédentes sur le cuir chevelu d'un individu.